(19) Europäisches Patentamt
European Patent Office
Office européen des brevets

(11) EP 3 457 849 B1

(12) **EUROPEAN PATENT SPECIFICATION**

(45) Date of publication and mention of the grant of the patent:
**29.07.2026 Bulletin 2026/31**

(21) Application number: **17733330.9**

(22) Date of filing: **19.06.2017**

(51) International Patent Classification (IPC):
***A01N 33/12*** (2006.01) ***A01N 43/40*** (2006.01)
***A01N 25/04*** (2006.01) ***A01N 33/04*** (2006.01)
***A01P 1/00*** (2006.01) ***A61L 2/22*** (2006.01)
***A61P 17/00*** (2006.01) ***A61P 31/04*** (2006.01)
***A61P 31/10*** (2006.01) ***A61P 31/12*** (2006.01)
***A61P 43/00*** (2006.01) ***A61L 2/18*** (2026.01)

(52) Cooperative Patent Classification (CPC):
(C-Sets available)
**A01N 33/12; A61L 2/18; A61L 2/22; A61P 17/00; A61P 31/04; A61P 31/10; A61P 31/12; A61P 43/00**
(Cont.)

(86) International application number:
**PCT/US2017/038095**

(87) International publication number:
**WO 2017/222963 (28.12.2017 Gazette 2017/52)**

(54) **SYNERGISTIC ANTIMICROBIAL COMBINATIONS CONTAINING QUATERNARY AMMONIUM BIOCIDE**

SYNERGISTISCHE ANTIMIKROBIELLE ZUSAMMENSETZUNGEN ENTHALTEND QUATERNÄRAMMONIUMBIOCID

COMBINATIONES SYNERGISTIQUES ANTIMICOBIENNES CONTENANT D'AMMONIUM QUATERNAIRE EN TANT QUE BIOCIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2016 US 201662354425 P**

(43) Date of publication of application:
**27.03.2019 Bulletin 2019/13**

(73) Proprietor: **Arxada, LLC**
**Morristown, NJ 07960 (US)**

(72) Inventors:
- **MCGEECHAN, Paula**
**Morristown, New Jersey 07960 (US)**
- **PREUSS, Andrea**
**Morristown, New Jersey 07960 (US)**

(74) Representative: **Michalski Hüttermann & Partner mbB**
**Kaistraße 16A**
**40221 Düsseldorf (DE)**

(56) References cited:
**WO-A1-00/65911** **WO-A1-95/12976**
**WO-A2-2011/116775** **CA-A- 1 164 338**
**JP-A- H02 184 604** **US-A- 6 080 789**
**US-A1- 2005 053 516** **US-A1- 2009 023 790**
**US-A1- 2015 282 480**

- **PONS J-L ET AL: "EVALUATION OF ANTIMICROBIAL INTERACTIONS BETWEEN CHLORHEXIDINE, QUATERNARY AMMONIUM COMPOUNDS, PRESERVATIVES AND EXCIPIENTS", JOURNAL OF APPLIED BACTERIO, BLACKWELL PUBLISHING LTD., OXFORD, GB, vol. 73, 1 January 1992 (1992-01-01), pages 395 - 400, XP009032023, ISSN: 0021-8847**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LONZA: "BARDAC (TM) 205M BARDAC (TM) 208M Quaternary Ammonium Compounds EPA Registered", HTTP://BIO.LONZA.COM/UPLOADS/TX_MWAXMARKETINGMATERIAL/LONZA_PRODUCTDATASHEETS_BARDAC_208M_PDS.PDF, 29 July 2007 (2007-07-29), pages 1 - 2, XP055191139, Retrieved from the Internet <URL:http://bio.lonza.com/uploads/tx_mwaxmarketingmaterial/Lonza_ProductDataSheets_Bardac_208M_PDS.pdf> [retrieved on 20150522]**
- **WEIGERT: "neodisher Dekonta AF - technical information", WWW.DRWEIGERT.COM, 27 May 2015 (2015-05-27), pages 1 - 2, XP055393090, Retrieved from the Internet <URL:https://www.drweigert.com/de/uploads/tx_product_manager/downloads/product/dataSheet/neodisher-Dekonta-AF_MB_de_PN4018_2015-05-01.pdf> [retrieved on 20170721]**



(52) Cooperative Patent Classification (CPC): (Cont.)

C-Sets
**A01N 33/12, A01N 25/04, A01N 33/04, A01N 33/12, A01N 43/40**

## Description

### RELATED APPLICATIONS

The present application is based on and claims priority to U.S. Provisional Patent application Serial No. 62/354,425, which was filed on June 24, 2016.

### BACKGROUND

A disinfectant refers to any chemical agent capable of killing, destroying, or inhibiting the growth of organisms, particularly microorganisms. Disinfectant products include hard surface cleaners, hand sanitizers, pre-disinfectant cleaners for instruments, sterilizing and high-level disinfectant compositions, and the like.

Ideally, a disinfectant has broad-spectrum activity against all types of microorganisms at various pH levels. The disinfectant should also have high efficacy so that a minimum amount of the anti-microbial agent can be used to save cost and to avoid or reduce any possible adverse effects caused by the anti-microbial agent. Also, it is desirable that the disinfectant is stable to any changes in temperature encountered during manufacturing, packaging, and shipping as well as during storage. Further, an ideal disinfectant is physically and chemically compatible with ingredients of different application systems so that the anti-microbial agent can suitably be incorporated in various products.

In the past, various different disinfectants have been suggested. For example, disinfectants that have been used in the past include alcohols such as isopropyl alcohol and ethanol, copper compounds, silver compounds, aldehydes, oxidizing agents such as sodium hypochlorite, and the like.

In addition to the above disinfectants, quaternary compounds have also been used in the past. Quaternary compounds, however, can have a limited activity spectrum. In addition, in certain applications, quaternary compounds need to be used at relatively high concentrations in order to achieve desired efficacy. Adding relatively high concentrations of a quaternary compound to a product, however, can be problematic. For instance, not only is the quaternary compound expensive to manufacture and produce but can also be subject to various regulatory requirements. Consequently, a need exists for a method for increasing the efficacy spectrum of quaternary compounds and simultaneously reducing the required concentration of the compounds while still maintaining the desired effect. In this regard, the present disclosure is directed to improved anti-microbial compositions containing a quaternary compound and to methods for using the compositions.

Biocidal compositions are inter alia known from US 2005/053516 A1, CA 1 164 338 A, WO 2011/116775 A2, WO 00/65911 A1, US 6 080 789 A, WO 95/12976 A1, US 2015/282480 A1, and US 2009/023790 A1. Further biocidal compositions are inter alia known from LONZA: "BARDAC (TM) 205M BARDAC (TM) 208M Quaternary Ammonium Compounds EPA Registered", (2007-07-29), pages 1 - 2, http://bio.lonza.com/uploads/tx_mwaxmarketingmaterial/Lonza_ProductData Sheet s_Bardac_208M_PDS.pdf and WEIGERT: "Neodisher Dekonta AF - technical information", pages 1 - 2, https://www.drweigert.com/de/uploads/tx_product_manager/downloads/product/dat aSheet/neodisher-Dekonta-AF_MB_de_PN4018_2015-05-01.pdf JP H02 184604 A discloses a germicidal composition containing 1 pt.wt. of compound expressed by the formula

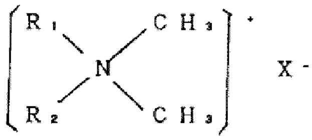

(1)

(R1 and R2 are octyl or decyl; $X^-$ is anionic counter ion) and 0.1-5 pts.wt. surfactant or further 0.2-5 pts.wt. nonionic surfactant. At least one selected from cetyltrimethylammonium salts, alkyldimethylbenzylammonium salts, monoalkylpolyaminoethylglycine salts and polyalkylpolyaminoethylglycine salts is used as the surfactant and examples of the nonionic surfactant include polyoxyethylene alkyl ethers.

PONS J-L ET AL: "EVALUATION OF ANTIMICROBIAL INTERACTIONS BETWEEN CHLORHEXIDINE, QUATERNARY AMMONIUM COMPOUNDS, PRESERVATIVES AND EXCIPIENTS", JOURNAL OF APPLIED BACTERIOLOGY, vol. 73, (1992-01-01), pages 395 - 400 discloses antimicrobial interactions of 49 combinations of chlorhexidine, quaternary ammonium compounds, preservatives and excipients, evaluated by the method of Berenbaum and the checkerboard titration method, with Staphylococcus aureus CIP 53154 and Escherichia coli CIP 54127 as test strains.

### SUMMARY

The invention relates to a composition having biocidal properties comprising: as a first biocide a didecyl dimethyl

ammonium carbonate/bicarbonate, and as a second biocide: a benzyl alkyl dimethyl ammonium chloride and/or an N,N-didecyl-N-methyl-poly(oxyethyl) ammonium propionate wherein the first biocide and the second biocide are present in the composition at a weight ratio of from about 1:20 to about 20:1. Combinations with further second biocides not being part of the present invention are also disclosed below. The second biocide synergistically operates in conjunction with the first biocide in order to kill or inhibit the growth of a microorganism or a plurality of different microorganisms. As used herein, a synergistic interaction refers to the fact that the two biocides combined together have a total effect that is greater than the biocide properties of only one of the biocides acting alone. In other words, the first and second biocides of the present invention operate synergistically together so as to have greater antimicrobial activity in the presence of each against a certain microorganism than in comparison to the antimicrobial activity of the first biocide alone or the antimicrobial activity of the second biocide alone at the same concentrations. Due to the synergistic effect, the amount of the first biocide present in the composition can be reduced while still producing the desired efficacy.

The second biocide is present in the composition in relation to the first biocide such that the ability of both biocides to kill or inhibit the growth of the microorganism is greater than if only one of the biocides were present at the same concentration of both biocides together.

Various different microorganisms may be killed in accordance with the present invention. For instance, the antimicrobial composition of the present invention can control gram positive bacteria, gram negative bacteria, fungi, yeast, spores, viruses, and the like. Examples of particular microorganisms that may be controlled in accordance with the present disclosure include *Escherichia coli, Staphylococcus aureus, Pseudomonas aeruginosa,* or mixtures thereof.

In one embodiment, the second biocide is benzyl alkyl dimethyl ammonium chloride, such as a benzyl $C_{12}$ to $C_{16}$ alkyl dimethyl ammonium chloride. In one particular embodiment, the composition may comprise a second biocide and a third biocide where the second biocide comprises benzyl alkyl dimethyl ammonium chloride and a third didecyl dimethyl ammonium chloride.

In another embodiment, the second biocide comprises N,N-didecyl-N-methyl-poly(oxyethyl)ammonium propionate.

In one embodiment not part of the present invention, the second biocide may comprise an amine, particularly a tertiary amine. One example of a tertiary amine that may be used in accordance with the present disclosure is N-3-aminopropyl-N-dodecylpropane-1-3-diamine.

In one embodiment not part of the present invention, the second biocide may comprise a pyrithione. For instance, a pyrithione salt may be used, such as sodium pyrithione.

In general, the first biocide and the second biocide are present in the composition at a weight ratio of from about 1:20 to about 20:1, such as from about 1:5 to about 5:1.

The anti-microbial composition of the present invention can be used in numerous and different products. For instance, the anti-microbial composition can be incorporated into a hard surface cleaner, a hand sanitizer, a disinfectant solution for instruments such as medical instruments, or in any other suitable disinfectant product. In addition to disinfectants, the anti-microbial composition of the present invention can also be used as a preservative composition and may be contained, for instance, in a cosmetic or hygiene product. The cosmetic or hygiene product may comprise a base composition and a preservative, wherein the preservative comprises the combination of biocides as described above.

The present disclosure is also directed to a method for producing a disinfectant composition. The method includes combining a first biocide with a second biocide that is selected so as to synergistically operate with the first biocide to kill or inhibit the growth of the microorganism. The second biocide can be any of the biocides described above.

Other features and aspects of the present disclosure are discussed in greater detail below.

## DETAILED DESCRIPTION

It is to be understood by one of ordinary skill in the art that the present discussion is a description of exemplary embodiments only, and is not intended as limiting the broader aspects of the present disclosure.

In general, the present invention is directed to a composition having biocidal properties. The composition has numerous uses and applications. In one embodiment, the composition may comprise a disinfectant. The disinfectant can be used in any suitable industry or field. For instance, the disinfectant can be used in the food and beverage field, may comprise an institutional product, a domestic product, or a healthcare product. The disinfectant, for instance, may comprise a hard surface disinfectant, a hand sanitizer, a sterilizing or high-level disinfectant composition, a pre-disinfectant cleaner for instruments and the like. In an alternative embodiment, the composition may be used as a preservative for a product, such as a personal care product or a hygiene product.

The composition of the present invention contains as a first biocide a didecyl dimethyl ammonium carbonate/-bicarbonate, and as a second biocide: a benzyl alkyl dimethyl ammonium chloride and/or an N,N-didecyl-N-methyl-poly(oxyethyl) ammonium propionate wherein the first biocide and the second biocide are present in the composition at a weight ratio of from about 1:20 to about 20:1. Combinations with further second biocides not being part of the present

invention are also disclosed below. The first biocide is combined with a second biocide to kill and/or control one or more microorganisms. The first biocide and the second biocide in accordance with the present invention operate synergistically together in order to kill or inhibit the growth of the microorganism. More particularly, the two biocides combined together in accordance with the present invention have greater efficacy at a lower concentration than if one of the biocides were present alone in the composition. The use of two different biocides also can enhance the spectrum of activity of the composition against multiple microorganisms. Thus, the anti-microbial composition of the present invention can be used at lower concentrations and yet be more effective against a wider range of microorganisms. Selection of the second biocide to be used in conjunction with the first biocide can be based upon the target microorganism and can be selected using a screening procedure. Ultimately, an anti-microbial composition can be produced with a total concentration of biocides that is relatively low while still providing robust preservation and/or antimicrobial properties.

The synergistic combination of biocides combined together in accordance with the present invention includes at least a first biocide and a second biocide. In some embodiments, a third biocide and/or a fourth biocide may be present to further enhance the synergistic effects. The present invention is directed particularly to selecting a second biocide that has synergistic interactions with the first quaternary ammonium salt. In this manner, the amount of the first quaternary ammonium salt contained in the composition can be minimized. In addition, due to the synergistic interactions, the amount of the second biocide can also be minimized. In other words, compositions according to the present invention contain two biocides having synergistic interactions wherein both biocides are contained in the composition at a total concentration less than if only one or more quaternary ammonium salts were present in the composition while still having the same or better efficacy against one or more target microorganisms.

A quaternary ammonium carbonate can be represented by the following formula:

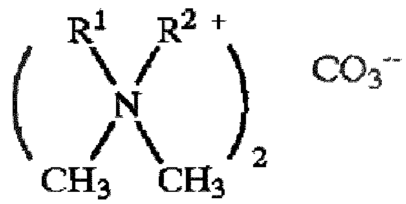

wherein $R^1$ is a $C_1$-$C_{20}$ alkyl or aryl-substituted alkyl group and $R^2$ is a $C_8$-$C_{20}$ alkyl group, and preferably wherein $R^1$ is the same as $R^2$ and $R^1$ is a $C_8$-$C_{12}$ alkyl group, as well as compositions further comprising the corresponding quaternary ammonium bicarbonate

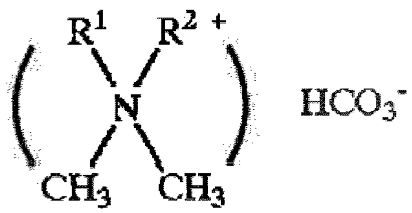

wherein $R^1$ is the same or a different $C_1$-$C_{20}$ alkyl or aryl-substituted alkyl group as above and $R^2$ is the same or a different $C_8$-$C_{20}$ alkyl group as above, but preferably wherein $R^1$ is the same as $R^2$ and $R^1$ is a $C_8$-$C_{12}$ alkyl group.

According to the present invention, the antimicrobial or preservative composition contains didecyl dimethyl ammonium carbonate and didecyl dimethyl ammonium bicarbonate.

In other embodiments which are not part of the present invention, however, the carbonate/bicarbonate salts of quaternary ammonium cations may be selected from dioctyldimethylammonium carbonate, decyloctyldimethylammonium carbonate, benzalkonium carbonate, benzethonium carbonate, stearalkonium carbonate, cetrimonium carbonate, behentrimonium carbonate, dioctyldimethylammonium bicarbonate, decyloctyldimethylammonium bicarbonate, benzalkonium bicarbonate, benzethonium bicarbonate, stearalkonium bicarbonate, cetrimonium bicarbonate, behentrimonium bicarbonate, and mixtures of one or more such carbonate salts.

In another embodiment not part of the present invention, the first biocide may comprise a quaternary ammonium halide. The quaternary ammonium halide may comprise, for instance, an alkyl quaternary ammonium halide or a benzyl ammonium halide.

Quaternary ammonium compounds, also known as "quats", typically comprise at least one quaternary ammonium cation with an appropriate anion. Quats will generally have the general formula (1).

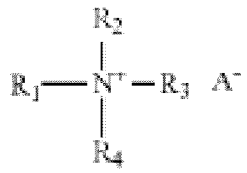

The groups $R_1$, $R_2$, $R_3$ and $R_4$ can vary within wide limits and examples of quaternary ammonium compounds that have anti-microbial properties will be well known to the person of ordinary skill in the art. Typically, two of $R_1$, $R_2$, $R_3$ and $R_4$ are lower alkyl, meaning having 1 to 4 carbon atoms, such as methyl, ethyl, propyl or butyl groups. In addition, two of $R_1$, $R_2$, $R_3$ and $R_4$ are longer chain alkyl groups of 6 to 24 carbon atoms, or a benzyl group. $A^-$ is a monovalent anion or one equivalent of a polyvalent anion of an inorganic or organic acid. Suitable anions for $A^-$ are in principle all inorganic or organic anions, in particular halides, for example chloride or bromide, carboxylates, sulfonates, phosphates or a mixture thereof.

In one embodiment not part of the present invention, the quaternary ammonium compound may have the following R groups: $R^1$ is benzyl or $C_{6-18}$-alkyl, $R_2$ is $C_{1-18}$-alkyl or $-[(CH_2)_2-O]_nR_5$ where n=1-20, $R_3$ and $R_4$ independently of one another are $C_{1-4}$-alkyl, $R_5$ is hydrogen or unsubstituted or substituted phenyl, and $A^-$ is a monovalent anion or one equivalent of a polyvalent anion of an inorganic or organic acid.

In one embodiment not part of the present invention, the quaternary ammonium compound may comprise a dialkyl ammonium compound, such as a dimethyl dialkyl ammonium compound. In one embodiment, the dimethyl dialkyl ammonium compound may have between about 8 and about 12 carbon atoms, such as from about 8 to about 10 carbon atoms in each of the alkyl groups.

Examples of dimethyl dialkyl ammonium compounds which may be used as the first biocide in embodiments not part of the present invention include dimethyl dioctyl ammonium compounds such as dimethyl dioctyl ammonium chloride, dimethyl didecyl ammonium compounds such as dimethyl didecyl ammonium chloride and the like. Mixtures of dimethyl dialkyl ammonium compounds may also be used and other anions, such as those described above may also be used. Commercially available dimethyl dialkyl ammonium compounds include, for example, compositions marketed and sold under the BARDAC™ tradename by Lonza America, Inc.

In an alternative embodiment not part of the present invention, the first biocide may comprise a benzyl ammonium compound, such as an alkyl dimethyl benzyl ammonium compound. In general, the alkyl group may contain from about 10 to about 18 carbon atoms, such as from about 12 to about 16 carbon atoms.

Examples of alkyl dimethyl benzyl ammonium compounds useable as the first biocide in embodiment not part of the present invention include $C_{12}$ alkyl dimethyl benzyl ammonium chloride, $C_{14}$ alkyl dimethyl benzyl ammonium chloride, and $C_{16}$ alkyl dimethyl benzyl ammonium chloride. In addition, a mixture of these alkyl dimethyl benzyl ammonium compounds can be used. Commercially available alkyl dimethyl benzyl ammonium compounds include, for example, compositions marketed and sold under the BARQUAT® tradename by Lonza America, Inc. These commercially available alkyl dimethyl benzyl ammonium compounds are blends of $C_{12}$, $C_{14}$, and $C_{16}$ alkyl dimethyl benzyl ammonium chlorides. Generally, it is preferable that the alkyl dimethyl benzyl ammonium compound, when a blend, contains higher concentrations of $C_{12}$ alkyl and $C_{14}$ alkyl components than $C_{16}$ alkyl components. It is noted that other anions, including those mentioned above, may also be used.

In still another embodiment not part of the present invention, the first biocide may comprise a quaternary ammonium propionate. The quaternary ammonium propionate, for instance, may comprise a poly(oxyalkyl)ammonium propionate. In one particular embodiment, for instance, the first biocide may comprise N,N-didecyl-N-methyl-poly(oxyethyl)ammonium propionate.

The first biocide as described above is combined with at least a second biocide. A second biocide is selected that synergistically operates with the first biocide to destroy and/or inhibit the growth of a target microorganism or a plurality of target microorganisms. In one embodiment, a second biocide is selected that is capable of increasing the efficacy of the first biocide against a microorganism such that the overall concentration of biocides in the composition can be reduced in comparison to a composition that only contains one of the biocides. As will be explained in the examples below, the second biocide can be selected through a screening process to determine synergistic interactions. The second biocide may comprise various different compounds depending upon the particular application and the microorganisms that are to be controlled.

In one embodiment, a second biocide is selected such that the sum of the fractional inhibitory concentrations of the first biocide and the second biocide are less than 1 when tested against a target microorganism. The fractional inhibitory concentration of a biocide is calculated as the concentration of a biocide which controlled growth in a mixture divided by the amount of biocide required to control growth when used alone. The fractional inhibitory concentration of a biocide can be calculated by dividing the concentration of the biocide attributable to antimicrobial activity in a mixture of the first biocide and the second biocide divided by the minimum inhibitory concentration of the biocide when tested against the target microorganism. In accordance with the present disclosure, when targeting a particular microorganism, the sum of

the fractional inhibitory concentrations of the first biocide and the second biocide can be less than about 0.9, such as less than about 0.8, such as less than about 0.7. Any value less than 1 indicates synergistic interactions.

In one embodiment not part of the present invention, the second biocide may comprise an amine. Amines, for instance, have been found to have a synergistic interaction with a quaternary ammonium carbonate when killing and controlling the growth of bacteria, particularly gram negative bacteria.

Suitable amines not part of the present invention include, but are not limited to, tertiary amines, such as ($C_8$-$C_{14}$) alkyl amines. The term "($C_8$-$C_{14}$) alkyl amine" encompasses all amines which contain a ($C_1$-$C_{14}$) alkyl group. One ($C_8$-$C_{14}$) alkyl amine is N,N-bis(3-aminopropyl)dodecylamine, available as Lonzabac® 12.30 and 12.100 from Lonza, Inc.

Other exemplary tertiary amines that are not part of the present invention include, for example, N-(3-aminopropyl)-N-dodecyl propane-1,3-diamine, N-(3-aminopropyl)-N-decyl-1,3-propanediamine, N-(3-aminopropyl)-N-tetradecyl-1,3-propanediamine as well as their acid addition compounds. Other similar tertiary amines may be used.

In yet another embodiment not part of the present invention, the second biocide present in the antimicrobial or preservative composition may comprise a pyrithione. The combination of a pyrithione and the first biocide has been found to have synergistic interactions when killing and controlling various microorganisms, such as bacteria and particularly when controlling gram positive bacteria.

Pyrithione is known by several names, including 2 mercaptopyridine-N-oxide; 2-pyridinethiol-1-oxide (CAS Registry No. 1121-31-9); 1-hydroxypyridine-2-thione and 1 hydroxy-2(1H)-pyridinethione (CAS Registry No. 1121-30-8). Pyrithione salts are commercially available from Lonza, Inc., such as Sodium OMADINE or Zinc OMADINE.

According to one embodiment not part of the present invention, the pyrithione present in the antimicrobial composition can be present in a water insoluble form or in a water soluble form. The pyrithione may comprise sodium pyrithione, zinc pyrithione, barium pyrithione, strontium pyrithione, copper pyrithione, cadmium pyrithione, and/or zirconium pyrithione. Other pyrithiones that may be present in the composition include sodium pyrithione, bismuth pyrithione, potassium pyrithione, lithium pyrithione, ammonium pyrithione, calcium pyrithione, magnesium pyrithione, silver pyrithione, gold pyrithione, manganese pyrithione, and/or an organic amine pyrithione. A single pyrithione may be present in the composition or a combination of any of the above may be included.

According to one embodiment not part of the present invention, the pyrithione particles can have a particle size such that 100% of the particles have a particle size of less than about 10 microns and at least 70% of the particles have a particle size less than 5 microns, such as at least about 50% of the particles can have a particle size of 1 micron or less. Particle size can be measured using a laser scattering particle size analyzer, such as a HORIBA LA 910 particle size analyzer.

According to one embodiment not part of the present invention, the pyrithione particles can be produced by reacting pyrithione or a water-soluble salt of pyrithione, and a water-soluble polyvalent metal salt in a pressurized, turbulent flow reactor that generates pulverizing forces. The pulverizing forces produced by the pressurized, turbulent flow reactor efficiently generate pyrithione salt particles of micron size. The micron-sized pyrithione salt particles made by the method have a narrow and uniform size distribution, and have excellent surface deposition properties due to the large surface area provided by the population of micron particles.

In one embodiment of the present invention the composition includes didecyl dimethyl ammonium carbonate and didecyl dimethyl ammonium bicarbonate in combination with a second biocide, such as didecylmethylpoly(oxyethyl)-ammonium propionate. The composition may further optionally include an alkalinity builder, a surfactant, a complexing agent, and/or a corrosion inhibitor. In a non-limiting embodiment, the optional alkalinity builder may be chosen from monoethanolamine (MEA), diethanolamine, triethanolamine, and mixtures thereof. The optional surfactant may be chosen from the group including but not limited to alkyl polyglucoside, cocamindopropyl betaine, cocamidopropyl hydroxylsultaine, alcohol polyglycolether, amine ethoxylate, fatty alcohol alkoxylate, polyethylene glycol, alcohol PEG-ether, and combinations thereof. The optional complexing agent may be chosen from but not limited to trisodium methylglycinediacetic acid, tetrasodium ethylenediaminetetraacetic acid, and mixtures thereof. The optional corrosion inhibitor may be chosen from but not limited to methyl dihydrogen phosphate, TEA neutralized polycarboxylic acid, and mixtures thereof.

In the above embodiment, the composition of the present invention may contain the first biocide in an amount from 3% to 15% by weight, such as from 4% to 10% by weight. The composition may contain the second biocide in an amount from 3% to 15% by weight, such as from 6% to 12% by weight.

In one embodiment, the composition may contain an alkalinity builder in an amount from 0.1% to 8% by weight, such as from 1% to 5% by weight.

In one embodiment, the composition may contain at least one surfactant in an amount from 0.1% to 8% by weight, such as from 0.5% to 6% by weight.

In one embodiment, the composition may contain a complexing agent in an amount from 0.1% to 5% by weight, such as from 0.25% to 3% by weight.

In one embodiment, the composition may contain a corrosion inhibitor in an amount from 0.1% to 5% by weight, such as from 0.25% to 3% by weight.

In one embodiment, the composition of the present invention comprises a didecyl dimethyl ammonium carbonate

and didecyl dimethyl ammonium bicarbonate, a didecylmethylpoly(oxyethyl)-ammonium propionate, an alkalinity builder, a surfactant, a complexing agent, and a corrosion inhibitor. The didecyl dimethyl ammonium carbonate and didecyl dimethyl ammonium bicarbonate may be present in the composition in an amount from about 3% by weight to about 15% by weight. The didecylmethylpoly(oxyethyl)-ammonium propionate may be present in the composition in an amount from about 3% by weight to about 15% by weight. The alkalinity builder may present in the composition in an amount from about 0.1% by weight to about 8% by weight. The surfactant may be present in the composition in an amount from about 0.1% by weight to about 8% by weight. The complexing agent may be present in the composition in an amount from about 0.1% by weight to about 5% by weight. The corrosion inhibitor may be present in the composition in an amount from about 0.1% by weight to about 5% by weight.

In another embodiment not part of the present invention, the composition of the present disclosure may include didecyl dimethyl ammonium carbonate and didecyl dimethyl ammonium bicarbonate in combination with a second biocide, such as N,N-bis(3-aminopropyl)dodecylamine. The composition may further optionally include an alkalinity builder, a surfactant, and/or a complexing agent. In a non-limiting embodiment, the optional alkalinity builder may be chosen from monoethanolamine (MEA), diethanolamine, triethanolamine, and mixtures thereof. The optional surfactant may be chosen from the group including but not limited to polyethylene glycol, alcohol PEG-ether, and combinations thereof. The optional complexing agent may be chosen from but not limited to trisodium methylglycinediacetic acid, tetrasodium ethylenediaminetetraacetic acid, and mixtures thereof.

In the above embodiment not according to the present invention, the composition of the present disclosure may contain the first biocide in an amount from 5% to 20% by weight, such as from 8% to 18% by weight. The composition may contain the second biocide in an amount from 1% to 10% by weight, such as from 2% to 8% by weight.

In one embodiment not according to the present invention, the composition may contain an alkalinity builder in an amount from 3% to 12% by weight, such as from 5% to 10% by weight.

In one embodiment not according to the present invention, the composition may contain at least one surfactant in an amount from 1% to 8% by weight, such as from 3% to 7% by weight.

In one embodiment not according to the present invention, the composition may contain a complexing agent in an amount from 2% to 10% by weight, such as from 3% to 8% by weight.

In one embodiment not according to the present invention, the composition may comprise a first biocide comprising a didecyl dimethyl ammonium carbonate and didecyl dimethyl ammonium bicarbonate, a N,N-bis(3-aminopropyl)dodecylamine, an alkalinity builder, a surfactant, and a complexing agent. The didecyl dimethyl ammonium carbonate and didecyl dimethyl ammonium bicarbonate may be present in the composition in an amount from about 5% by weight to about 20% by weight. The N,N-bis(3-aminopropyl)dodecylamine may be present in the composition in an amount from about 1% by weight to about 10% by weight. The alkalinity builder may present in the composition in an amount from about 3% by weight to about 12% by weight. The surfactant may be present in the composition in an amount from about 1% by weight to about 8% by weight. The complexing agent may be present in the composition in an amount from about 2% by weight to about 10% by weight.

The composition of the present invention can be first formulated into a concentrate which can then be diluted and added to a final product. The relative amounts of the different components can vary significantly based on many factors. The factors include the type of biocides used, the end use application, and the desired microorganism to be controlled. The first biocide is present in relation to the second biocide at a weight ratio of from about 20:1 to about 1:20, such as from about 1:5 to about 5:1.

As described above, the first and the second biocide can be contained together in a concentrate and later diluted when used. When producing a disinfectant product, for instance, the first and second biocide can be present in the concentrate in an amount greater than about 5% by weight, such as in an amount greater than about 10% by weight, such as in an amount greater than about 15% by weight, such as in an amount greater than about 20% by weight. The concentrate can contain the first and second biocide in an amount less than about 50% by weight, such as in an amount less than about 30% by weight. During use, the concentrate can be diluted such that the final product contains the first and second biocide in an amount greater than about 0.01% by weight, such as in an amount greater than about 0.1% by weight, such as in an amount greater than about 0.5% by weight, such as in an amount greater than about 0.75% by weight, such as in an amount greater than about 1% by weight, such as in an amount greater than about 1.25% by weight, such as in an amount greater than about 1.5% by weight. The final product generally contains the first and second biocide in an amount less than about 5% by weight, such as in an amount less than about 3% by weight, such as in an amount less than about 2% by weight.

When the first and second biocide are used as a preservative, the preservative in concentrated form can contain the first and second biocide in an amount greater than about 15% by weight, such as in an amount greater than about 20% by weight, such as in an amount greater than about 25% by weight, such as in an amount greater than about 30% by weight, such as in an amount greater than about 35% by weight. The concentrated preservative composition generally contains the first and second biocide in an amount less than about 50% by weight, such as in an amount less than about 45% by weight, such as in an amount less than about 40% by weight. When added to a product as a preservative, the concentrate is

then diluted such that the preservative contains the first and second biocide in an amount greater than about 0.01% by weight, such as in an amount greater than about 0.05% by weight, such as in an amount greater than about 0.1% by weight, such as in an amount greater than about 0.2% by weight, such as in an amount greater than about 0.3% by weight, such as in an amount greater than about 0.4% by weight, such as in an amount greater than about 0.5% by weight. The first and second biocide can be contained in the preservative when added to a product in an amount less than about 2% by weight, such as in an amount less than about 1.5% by weight, such as in an amount less than about 1% by weight. It should be understood, however, that the above ranges are merely exemplary.

Various different microorganisms may be killed or controlled in accordance with the present invention. For instance, the antimicrobial composition of the present invention can control gram positive bacteria, gram negative bacteria, and the like. In addition to bacteria, the anti-microbial composition of the present invention can also kill and control the growth of various other microorganisms, such as fungi, viruses, spores, yeast, mycobacteria, and the like. Examples of particular microorganisms that may be killed or controlled in accordance with the present disclosure include *Staphylococcus aureus, Streptococcus pneumoniae, Pseudomonas aeruginosa, Serratia marcescens, Salmonella enteritidis, Neisseria gonorrhoeae, Escherichia coli, Enterococcus hirae, Acinetobacter baumannii, Listeria monocytogenes, Enterobacter gergoviae, Klebsiella pneumoniae, Burholderia cepacia, Pseudomonas putida, Kocuria rhizophila, Candida albicans, Saccharomyces cerevisiae, Aspergillus brasiliensis, Penicillium funiculosum, Eupenicillium levitum, Bacillus cereus, Bacillus subtilis, Clostridium difficile, Clostridium perfringens, Mycobacterium tuberculosis, Mycobacterium terrae, Mycobacterium avium,* Poliovirus, Adenovirus, Norovirus, Vaccinia virus, Influenza virus, Hepatitis B virus, Human Immunodeficiency virus, Human papilloma virus, or mixtures thereof.

In addition to a first biocide and a second biocide, the composition can contain various other components and ingredients. For instance, the first and second biocide can be combined with various different components depending upon whether the biocides are being formulated into a disinfectant product or being used as a preservative.

*Disinfectant Applications*

Various different disinfectant compositions can be made in accordance with the present invention. The disinfectant product may be used, for instance, to clean hard surfaces, to pre-clean sterilize or high-level disinfect instruments, and/or as a hand sanitizer. In general, the first and second biocide can be incorporated into any suitable disinfectant product.

When used as a hard surface cleaner, the anti-microbial composition can be delivered to a surface to be cleaned, sanitized or disinfected by conventional means such as pouring the composition on a surface; a spray; which is applied to a surface via a spray means, including but not limited to, pump spray applicators, pressurized spray applicators and the like; a saturated wipe; a rag and a bucket; a mop and bucket; a sponge and a bucket; or via automated cleaning equipment and other similar and conventional ways to apply an anti-microbial composition to a surface for the purposes of sanitizing or disinfecting the surface.

To use the anti-microbial composition of the present disclosure, a surface is treated with the substrate by spraying, pouring, wiping or otherwise applying the anti-microbial composition to the surface. Once applied to the surface, the anti-microbial composition is allowed to remain on the surface for a period of time. The anti-microbial composition may be applied to the surface and allowed to dry or may alternatively be dried by wiping the surface with a dry wipe or wiping device.

Surfaces, which may be disinfected with the compositions include, but are not limited to, those located in dairies, homes, health care facilities, swimming pools, canneries, food processing plants, restaurants, hospitals, institutions, and industry, including secondary oil recovery. Hard surfaces, such as glass and polished aluminum, are particularly suited for application. Specific areas targeted for application include hard surfaces in the home such as kitchen countertops, cabinets, appliances, waste cans, laundry areas, garbage pails, bathroom fixtures, toilets, water tanks, faucets, mirrors, vanities, tubs, and showers. The compositions can also be used to sanitize floors, walls, furniture, mirrors, toilet fixtures, windows, and wood surfaces, such as fence rails, porch rails, decks, roofing, siding, window frames, and door frames. The compositions, quaternary ammonium chloride compound, and disinfecting active are particularly well suited for application on indirect food contact surfaces, such as cutting boards, utensils, containers, dishes, wash basins, appliances, and countertops. The compositions or quaternary ammonium chloride compound can be used to sanitize dairy plant equipment, milking machines, milk pails, tank trucks, and the like. Areas in hospitals would include beds, gurneys, tables, canisters, toilets, waste cans, stands, cabinets, shower stalls, floors, walls or any other non-porous surface.

In addition to a hard surface cleaner, as described above, the anti-microbial composition can also be incorporated into a hand sanitizer, a pre-disinfectant cleaner or a high-level disinfectant or sterilant solution.

*Disinfectant*

When contained in a disinfectant product, the biocides may be combined with various different components. For

instance, in one embodiment, a solvent can be present in the product. Generally, the solvent will be a polar solvent such as water, or a water-miscible solvent, such as an alcohol and/or a glycol ether. In addition to water, the anti-microbial composition can further include a water-miscible organic solvent. Examples of water-miscible solvents include ethanol, propanol, benzyl alcohol, phenoxyethanol, isopropanol, diethylene glycol propyl ether, ethylene glycol monomethyl ether, ethylene glycol monoethyl ether, ethylene glycol monopropyl ether, ethylene glycol monoisopropyl ether, ethylene glycol monobutyl ether, diethylene glycol monomethyl ether, diethylene glycol monobutyl ether, diethylene glycol monoethyl ether, diethylene glycol mono-n-butyl ether, ethylene glycol dimethyl ether, ethylene glycol diethyl ether, ethylene glycol dibutyl ether, propylene glycol n-butyl ether, tripropylene glycol methyl ether, dipropylene glycol methyl ether, dipropylene glycol butyl ether and combinations thereof.

In addition to a solvent, the disinfectant product may contain a surfactant. Typically, the surfactant will be a nonionic surfactant or a cationic surfactant. The surfactant is present in an amount between about 1 % to about 20% by weight of the disinfectant concentrate. Typically, the surfactant will be between 2% and 15% by weight of the concentrate.

Particularly suitable surfactants are alkoxylated alcohol surfactant will generally have between about 2 to about 8 moles of alkoxylation. Typically, there will be between 3 and 6 moles of alkoxylation. One particular example is about 4.5 moles of alkoxylation. In addition to having the degree of alkoxylation, the alcohol which is alkoxylate will be a Ce- C12 alkyl alcohol. In one embodiment, the alkyl alcohol is a Cs- C10 alkyl alcohol. The alkoxylation may be ethoxylation. Generally, it is desirable to have the HLB (hydrophilic-lipophilic balance) to be in the range of 8-14, and more generally between 10 and 12, for example about 11.

The non-ionic surfactants that may be used in the invention include, but are not limited to, polyoxyethylene glycol alkyl ethers, octaethylene glycol monododecyl ether, pentaethylene glycol monododecyl ether, polyoxypropylene glycol alkyl ethers, glucoside alkyl ethers, decyl glucoside, lauryl glucoside, octyl glucoside, polyoxyethylene glycol octylphenol ethers, polyoxyethylene glycol alkylphenol ethers, glycerol alkyl esters, polyglycerol esters, glyceryl laurate, polyoxyethylene glycol sorbitan alkyl esters, sorbitan alkyl esters, dodecyldimethylamine oxide, block copolymers of polyethylene glycol and polypropylene glycol, poloxamers and polyethoxylated tallow amine (POEA), and mixtures thereof. The amount of the nonionic surfactant in the concentrate is from about 1 to about 8% w/w % of the formulation. Typically, the concentrate contains from 2 to 5 w/w % of nonionic surfactant. The amount of the nonionic surfactant in the ready-to-use product is from about 0.05 to about 3 w/w % of the solution. In another embodiment, the nonionic surfactant in the product is from about 0.05 to about 1.5 w/w % of the solution. Typically, the disinfectant product contains from 0.06 to 1 w/w % of the nonionic surfactant.

Additionally, the disinfectant product may contain an optional sequestering agent. Sequestering agents include, for example, acetic acid derivative selected from the group consisting of ethylenediaminetetraacetic acid (EDTA), nitrilotriacetic acid (NTA), tetrasodium EDTA. The ability of NTA and EDTA to remove metal ions facilitates of the solution by preventing hardness (calcium) precipitation. The sequestering agent may also serve to bind other metal ions that may adversely affect the effectiveness of the disinfecting components in the composition. In addition, sequestering agent may also assist in soil removal and/or preventing soil redeposition into the disinfecting composition while in use. The sequestering agents, when present in the concentrate is generally present in an amount up to about 20% by weight, and are typically present in an amount of about 2 to about 8% by weight.

The disinfectant product may also contain a pH adjusting agent. Suitable pH adjusting agents include sodium hydroxide, sodium citrate and other similar compounds. In the present invention, the concentrate and the final disinfectant composition will have a pH in the range of about 6 to about 1 3. Generally the disinfectant composition will be considered a neutral disinfecting composition if the pH is in the range of about 6 to about 8. The disinfectant composition will be considered an alkaline disinfectant composition when the pH is in the range of above 8 to about 12.

The disinfectant product may optionally further contain corrosion inhibitors, complexing agents, auxiliaries, preservatives, fragrances, colorants and the like. Exemplary corrosion inhibitors include, for example, organic phosphorous compounds and blend of organic phosphorous compounds with a polymeric component. Exemplary auxiliaries include, for example, polyethylene glycol or other similar compounds. Colorants and fragrances may be added provided they do not interfere with the function of the composition and may serve for identifying the composition. Generally, the optional further ingredients will make up less than about 20% by weight of the composition.

The antimicrobial composition may also comprise at least one acid or salt thereof. The acid may be an inorganic acid or an organic acid. In a preferred embodiment the acid is a C1 to C8 carboxylic acid. In a more preferred embodiment, the acid is a monocarboxylic acid, a dicarboxylic acid, a tricarboxylic acid, or a mixture thereof. In an additional embodiment, the acid is a hydroxyacid, an aromatic acid, or a mixture thereof. In another additional embodiment, the acid is methanesulfonic acid, phosphoric acid, etidronic acid, phytic acid, phosphoacetic acid, N-(phosphonomethyl) iminodiacetic acid, diethylenetriaminepentakis(methylphosphonoic acid), S,S-ethylenediamine-N'N'-disuccinic acid, their alkaline salts, or any mixture thereof.

In some embodiments, the acid is citric acid, phosphoric acid, succinic acid, lactic acid, S,S-ethylenediamine-N,N'-disuccinic acid, 1-hydroxyethane 1,1-diphosphonic acid (HEDP), dipicolinic acid (DPA), methanesulfonic acid (MSA), their alkaline salts, or any mixture thereof.

In a preferred embodiment, the acid is a mixture of acids. In some embodiments, the acid comprises one or more of the following organic acids: citric acid, succinic acid, phosphoric acid, and lactic acid. In another embodiment, the acid comprises one or more of the following acids: citric acid, succinic acid, phosphoric acid, and lactic acid, in combination with another acid. For example, citric acid may be used in combination with ethylenediamine-N,N'-disuccinic acid or its alkaline salt, HEDP, and/or MSA. As another example, succinic acid may be used in combination with ethylenediamine-N,N'-disuccinic acid or its alkaline salt, HEDP, and/or MSA. As another example, phosphoric acid may be used in combination with ethylenediamine-N,N'-disuccinic acid or its alkaline salt, HEDP, and/or MSA. As another example, lactic acid may be used in combination with ethylenediamine-N,N'-disuccinic acid or its alkaline salt, HEDP, and/or MSA.

The composition may include from about 1% by weight to about 5% by weight of an organic acid such as citric acid, succinic acid, phosphoric acid, lactic acid, or any mixture thereof, in combination with another acid. In another aspect, the composition may include from about 1% by weight to about 5% by weight of an organic acid such as citric acid, succinic acid, phosphoric acid, lactic acid, or any mixture thereof, in combination with from about 0.05% by weight to about 5% by weight of another acid. In another embodiment, the composition may include from about 2% by weight to about 4% by weight of an organic acid such as citric acid, succinic acid, phosphoric acid, lactic acid, or any mixture thereof, in combination with from about 0.1% by weight to about 4% by weight of another acid such as ethylenediamine-N,N'-disuccinic acid or its alkaline salt, HEDP, and/or MSA.

One particularly useful application method is to impregnate the disinfectant composition into a wipe substrate. In this embodiment, the wipe is a single use wipe that is impregnated with the disinfecting composition and is stored in a container that will dispense the wipe to a user. The container with the wipes may contain a single wipe, or several wipes. Suitable containers include a pouch containing a single wipe, such as a moist towelette which is torn open by the user, or may be a pouch with a resealable opening containing several wipes in a stacked fashion, a rolled fashion or other suitable formation that would allow a single wipe to be removed from the opening at a time. Pouches are generally prepared form a fluid impervious material, such as a film, a coated paper or foil or other similar fluid impervious materials. In another way to dispense wipes of the present invention is to place the wipe in to a fluid impervious container having an opening to access the wipes in the container. Containers may be molded plastic container with lids that are fluid impervious. Generally, the lid will have an opening to access the wipes in the container. The wipe in the container may be in a interleaved stacked, such that as a wipe is removed from the container the next wipe is positioned in the opening of the container ready for the user to remove the next wipe. Alternatively, the wipe may be a continuous material which is perforated between the individual wipes of the continuous material. The continuous wipe material with perforations may be in a folded form or may be in a rolled form. Generally, in the rolled form, the wipe material is feed from the center of the rolled material. As with the interleaved stack, as a wipe is removed from the container, the next wipe is positioned in the opening for the use to remove the next wipe, when needed.

Disposable wipes provide advantages over other application vehicles, such as a reusable sponge, rag or the like. Unlike sponges, rags and the like, which are used repeatedly, the impregnated wipe is used a single time and disposed of. As is mentioned above, reused sponge or rag presents problems since the sponge or rags may carry microbes that are not easily killed by the disinfecting composition. Further, the disinfecting composition is formulated to treat hard surface, not porous soft surfaces that are present in sponges or rags.

The disinfecting composition can be impregnated into the wipe such that the wipe is pre-moistened and will express or release the disinfecting composition on to the surface as the wipe is run across the surface to be treated. Generally, the disinfecting composition is saturated into the wipe such that the wipe will release the disinfecting composition to the surface through the wiping action. Depending on the wipe substrate, saturation was generally achieved using about 3 wt parts of the use disinfecting composition per 1 wt part of the wipe substrate to be saturated. Generally, the disinfecting composition is used from about 4 parts to 6 parts by weight per 1 part by of the wiper substrate. In these ranges, complete saturation of the substrates can be achieved. I t is noted that the amount of the disinfecting solution may go up or down to achieve complete saturation of the wipe substrate, depending on the particular wipe substrate.

Suitable wipe substrates include woven and nonwoven materials. Essentially any nonwoven web material may be used. Exemplary nonwoven materials may include, but are not limited to meltblown, coform, spunbond, airlaid, hydroentangled nonwovens, spunlace, bonded carded webs, and laminates thereof. Optionally, the nonwoven may be laminated with a film material as well. The fibers used to prepare the wipe substrate may be cellulosic fiber, thermoplastic fibers and mixtures thereof. The fibers may also be continuous fibers, discontinuous fibers, staple fibers and mixtures thereof. Basis weights of the nonwoven web may vary from about 12 grams per square meter to 200 grams per square meter or more.

In one embodiment the wipe is impregnated with a liquid component containing both active and inert ingredients within the allowable tolerance levels and the disinfecting composition expressed from the wipe contains active ingredients within the allowable tolerance levels. Once applied to the surface, the antimicrobial disinfecting composition is allowed to remain on the surface for a period of time. The antimicrobial composition may be applied to the surface and allowed to dry or may alternatively be dried by wiping the surface with a dry wipe or wiping device, which is preferably unused.

When the wipe or disinfecting composition of the present invention is used to wipe a surface, disinfection is

achieved in less than 4 minutes, generally 3 minutes or less and specifically in 90 seconds or less. It will be understood by those of ordinary skill that the antimicrobial disinfecting composition remains in contact with the surface requiring disinfection for a time sufficient to cause disinfection to occur. It has been discovered that the composition of the present invention is effective against many different microbes, including, but not limited to.

In yet another embodiment, the disinfectant composition may be used as a hand sanitizer. When used as a hand sanitizer, the biocides of the present disclosure can be combined with any of the ingredients described above. In one embodiment, for instance, the biocides may be combined with a solvent, such as water and/or an alcohol. In one particular application, a foaming agent may be added that causes the composition to foam when pumped from a dispenser. The foaming agent may comprise any suitable foaming agent that is compatible with the biocides. In one embodiment, for instance, the foaming agent may comprise a dimethicone.

In one embodiment, the composition of the present disclosure may include a second biocide and a third biocide combined with the first biocide. For instance, in one embodiment not part of the present invention, the second biocide may comprise a dimethyl dialkyl ammonium compound as described above and the third biocide may comprise an aromatic dialdehyde. The aromatic dialdehyde may be chosen from orthophthalaldehyde (OPA), isophthalaldehyde, terephthalaldehyde, or combinations thereof. The structures of the phthalaldehyde isomers are shown below:

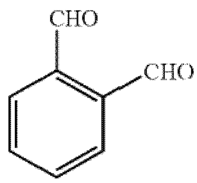

Orthophthalaldehyde
(OPA)

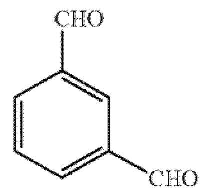

Isophthalaldehyde

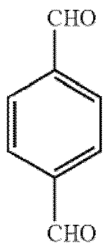

Terephthalaldehyde

In one embodiment, the preferred aromatic dialdehyde is OPA.

In one embodiment, the disinfectant composition may be used for disinfection of instruments, such as for precleaning and disinfection or for terminal, high-level disinfection of a device, medical instrument or endoscope. In one embodiment, when the instrument is treated in a manual process, the disinfectant composition could be applied by immersing the instrument in the appropriate concentration of the disinfectant composition. For instance, plastic or metal containers, stainless steel sinks, or any other suitable container may be used as a vessel to hold the disinfectant composition. In one embodiment, complete immersion of the instrument or device or endoscope, including voids, lumens and hollow sections, may be necessary When used for disinfection of instruments such as endoscopes, the channels of the endoscope and other instruments may need to be flushed. In general, after disinfection the instrument must be rinsed and flushed thoroughly with water, preferably with significant quantities of water.

In applications involving instruments, the appropriate concentration of the disinfectant composition may be from about 500 mg/L to about 25,000 mg/L, such as from about 1,000 mg/L to about 23,000 mg/L. For precleaning, the preferred concentration of the disinfectant composition may be from about 500 mg/L to about 10,000 mg/L, such as from about 1,000 mg/L to about 9,000 mg/L, such as from about 2,000 mg/L to about 8,000 mg/L, such as from about 3,000 mg/L to about 7,000 mg/L, such as from about 4,000 mg/L to about 6,000 mg/L. For high level disinfection, the preferred disinfectant composition concentration may be from about 5,000 mg/L to about 25,000 mg/L, such as from about 8,000 to about 23,000 mg/L, such as from about 10,000 to about 20,000 mg/L. In embodiments in which the instrument is immersed in the disinfectant composition, the the necessary contact time may range from about 10 minutes to about 60 minutes, preferably from about 15 minutes to about 30 minutes. The necessary contact time may be adjusted based on the targeted disinfection level. In yet another embodiment, the disinfectant composition may be used for the disinfection of instruments in an automated washer-disinfector.

*Preservative*

In addition to disinfectant compositions, the biocides of the present invention can also be used as a preservative. When used as a preservative, the biocides can be incorporated into any suitable product, such as a paint, a latex emulsion, a polymer emulsion, an adhesive, a sealant, a caulk, a mineral or pigment slurry, a printing ink, a pesticide formulation, a household product, a personal care product, a hygiene product, a metal working fluid, and the like. In addition, the preservative may be incorporated into a building material. Personal care products that may contain the preservative include a cosmetic formulation, such as a face cream, makeup remover, or mascara. The personal care product may also comprise shampoo, a conditioner, or a skin lotion.

When used as a preservative, the biocides may be combined with any suitable surfactant. The surfactant, for instance, may comprise an anionic surfactant.

Suitable anionic surfactants having a sulfate moiety include sulfates represented by the formula $RSO_4M$, wherein R is a chain containing from about 10 to about 18 atoms at the backbone of the chain, M is a cation such as ammonium; alkanolamines, such as triethanolamine; monovalent metals, such as sodium and potassium; and polyvalent metals, such as magnesium, and calcium. In one embodiment, the anionic surfactants having a sulfate moiety are alkyl sulfates wherein R is an alkyl having from 10 to 18 carbon atoms, preferably from 10 to 16 carbon atoms, more preferably from 10 to 14 carbon atoms. R can be a straight or branched chain. Advantageously, R is a straight chain. In one embodiment, R is an octyl group.

Exemplary alkyl sulfates include sodium dodecyl sulfate, potassium dodecyl sulfate, triethylamine dodecyl sulfate, triethanolamine dodecyl sulfate, monoethanolamine dodecyl sulfate, diethanolamine dodecyl sulfate and ammonium dodecyl sulfate.

Suitable anionic surfactants having a sulfonate moiety include sulfonates having the formula $RSO_3M$, wherein R is a straight or branch chain containing 10 to 18 atoms at the backbone of the chain, M is a cation such as ammonium; alkanolamines, such as triethanolamine; monovalent metals, such as sodium and potassium; and polyvalent metals, such as magnesium, and calcium. In one embodiment, R contains a succinate group at the backbone. In another embodiment, R is an alkyl group containing from about 10 to about 18 carbon atoms, preferably from about 10 to about 16 carbon atoms, more preferably, from about 10 to about 14 carbon atoms.

Exemplary sulfonates suitable for the composition of the invention includes sodium dioctyl sulphosuccinate, and primary and secondary alkyl sulphonates.

Suitable anionic surfactant (ii) is an alkylaryl sulfonic acid or salt thereof wherein the alkyl portion contains from about 10 to about 18 carbon atoms, and the aryl portion contains a benzyl or substituted benzyl group. In one embodiment, the alkylaryl sulfonic acid is represented by the formula

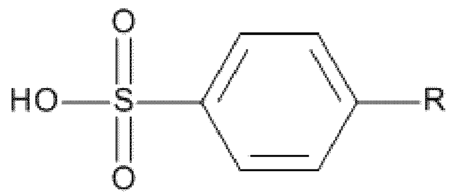

wherein R is an alkyl having from about 10 to about 18, preferably from 10 to 16, more preferably from 10 to 14 carbon atoms. In one embodiment, R is a C12 alkyl group.

Whether a surfactant is present may depend upon the end use application of the composition. In this regard, the composition (concentrate or final product) may contain and emulsifier, a consistency factor, an emollient, a thickener, a filler, a lubricant, a fragrance, and the like.

When incorporated into a personal care product, the product may contain an emulsifier, a consistency factor, an emollient, and/or an active ingredient.

Suitable emulsifiers are e.g. anionics as salts of fatty acids e.g. sodium stearate or sodium palmitate, organic soaps e.g. mono-, di- or triethanolaminoeate, sulfated or sulfonated compounds e.g. sodium lauryl sulfate or sodium cetyl sulfonate, saponines, lamepones; cationics as quaternary ammonium salts; nonionics as fatty alcohols, fatty acid ester with saturated or unsaturated fatty acids, polyoxyethylenesters or polyoxyethylenethers of fatty acids, polymers from ethylene oxide and propylene oxide or propylene glycol, amphotherics as phosphatides, proteins as gelatine, casein alkylamidobetaines, alkyl betaines and amphoglycinates, alkyl phosphates, alkylpolyoxyethylene phoaphates or the corresponding acids, silicone derivatives, e.g. alkyl dimethiconecoplyol.

Suitable consistency factors are e.g. fatty alcohols or their mixtures with fatty acid esters, e.g. acetylated lanolin alcohol, aluminum stearates, carbomer, cetyl alcohol, glyceryl oleate, glyceryl stearate, glyceryl stearate (and) PEG 100 stearate, magnesium stearate, magnesium sulfate, oleic acid, stearic acid, stearyl alcohol, myristyl myristate, isopropyl palmitate, beeswax and synthetic equivalents thereof, carbomers, and the like. Suitable conditioners are e.g. alkylamido

ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl palmitate, quaternized protein hydrolysates, quaternized cellulose and starch derivatives, quaternized copolymers of acrylic or methacrylic acid or salts, quaternized silicone derivatives.

Suitable emollients are e.g. cetearyl isononanoate, cetearyl octanoate, decyl oleate, isooctyl stearate, coco caprylate/caprate, ethylhexyl hydroxystearate, ethylhexyl isononanoate, isopropyl isostearate, isopropyl myristate, oleyl oleate, hexyl laurate, paraffinum liquidum, PEG-75 lanolin, PEG-7 glyceryl cocoate, petrolatum, ozokerite cyclomethicone, dimethicone, dimethicone copolyol, dicaprylyl ether, butyrospermum parkii, buxus chinensis, canola, carnauba cera, copernicia cerifera, oenothera biennis, elaeis guineensis, prunus dulcis, squalane, zea mays, glycine soja, helianthus annuus, lanolin, hydrogenated castor oil, hydrogenated coconut oil, hydrogenated polyisobutene, sucrose cocoate, stearoxy dimethicone, lanolin alcohol, isohexadecane.

The compositions may further contain active ingredients, e.g. antimicrobials, anti-inflammatories, plant extracts, bisabolol, panthenol, tocopherol, actives for anti-stinging, anti-irritant or anti-dandruff applications, or anti-aging agents such as retinol, melibiose and the like. Other suitable actives are e.g. Medicago officinalis, Actinidia chinensis, allantoin, Aloe barbadensis, Anona cherimolia, Anthemis nobilis, Arachis hypogaea, Arnica Montana, Avena sativa, beta-carotene, bisabolol, Borago officinalis, butylenes glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea Arabica, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glycerin, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, Lactis proteinum, lactose, Lawsonia inermis, linalool, Linum usitatissimum, lysine, magnesium aspartate, Magnifera indica, Malva sylvestris, mannitol, mel Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, Prunus armeniaca, Prunus persica, retinyl palmitate, Ricinus communis, Rosa canina, Rosmarinus officinalis, Rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl praline, stearoxytrimethylsilane, stearyl alcohol, sulfurized TEA-ricinoleate, talc, Thymus vulgaris, Tilia cordata, tocopherol, tocopheryl acetate, trideceth-9, triticum vulgare, tyrosine, undecylenoyl glycine, urea, Vaccinium myrtillus, valine, zinc oxide, zinc sulfate.

The present invention may be better understood with reference to the following examples.

## Examples

Various different biocides were tested for minimum inhibitory concentration against various microorganisms. More particularly, MIC tests were conducted against a quaternary ammonium carbonate/bicarbonate and various other biocides. The quaternary ammonium carbonate/bicarbonate was then combined with one of the other biocides and tested for synergistic interaction against various microorganisms. The results below demonstrate synergistic action of the quaternary ammonium carbonate/bicarbonate with the other biocides.

### MATERIALS

Sterile 96 well microtitre plates were obtained from Fisher scientific. Titretek self adhesive plate sealers were obtained from Fisher scientific. 50ml reservoirs obtained from Fisher scientific
Malt agar, malt broth, nutrient agar and nutrient broth were obtained from E&O.

### MICROBIAL STRAINS USED

| | | |
|---|---|---|
| Bacterial | *Escherichia coli* | ATCC 1576 |
| | *Staphylococcus aureus* | ATCC 799 |
| | *Pseudomonas aeruginosa* | ATCC 9027 |

**MAINTENANCE OF STOCK CULTURES**

Bacteria were maintained on nutrient agar and fungi on malt agar or a spore suspension in physiological saline (0.85% (w/v) NaCl).

**CALCULATION OF MINIMUM INHIBITORY CONCENTRATIONS**

Bacteria were grown to stationary phase (18h) in nutrient broth (approximately $10^9$ organisms per ml). A 0.1% (v/v) inoculum was used to seed fresh medium and 100μl of this was added to each well of a microtitre plate, except for the first well which contained 200μl. Using doubling dilutions, the concentration of each antimicrobial was varied in each well along the ordinate axis. The presence or absence of growth was determined by visual inspection after 24 hours incubation at 37°C.

**CALCULATION OF SYNERGY USING MICROTITRE PLATES**

A simple matrix was constructed with varied concentrations of the two compounds under investigation from 2 x MIC (Minimum Inhibitory Concentration) down to zero concentration in an 8 x 8 array. Solutions were made up in broth at two times the final concentrations required after pre-diluting the materials under test in deionised water.

Each solution (100μl) was added to the plate so that the total volume of each mixture in each well was 200μl. Nutrient broth was used for *E.coli* and *S.aureus* and malt broth for *C.albicans.* Plates were incubated for 16-24 hours at 37°C for bacteria, 48 hours for yeast and 40-72 hours at 25°C for fungi. The presence or absence of growth was recorded by visual inspection.

**RESULTS**

The Minimum Inhibitory Concentration (MIC) is the lowest concentration of biocide which showed growth inhibition when used alone. Results are shown in Table 1.

**Table 1: Minimum Inhibitory Concentrations**

| Product Name | Chemical Name | *MIC versus organism (ppm)* | | |
|---|---|---|---|---|
| | | ***E.c*** | ***S.a*** | ***Ps.a*** |
| Carboquat HE | Didecyl dimethyl ammonium carbonate/ bicarbonate | 4 | 1 | 8 |
| Bardac 22.70 | Didecyldimethyl ammonium chloride | 4 | 1 | 8 |
| Barquat DM-80 | Benzyl C12-16 alkyl dimethyl chlorides | 12 | 2 | 32 |
| Barquat 4214 | Alkyldimethylbenzylammonium chloride | 8 | 2 | 32 |
| Bardac 208M | di C8-10 alkyl dimethyl chlorides + Benzyl C12-16 alkyl dimethyl chlorides | 9 | 2 | 13 |
| Bardap 26 | N,N-Didecyl-N-methyl-poly(oxyethyl)ammonium propionate | 4 | 1 | 16 |
| Lonzabac 12.30 | N-3-aminopropyl-N-dodecylpropane-1-3-diamine | 16 | 8 | 32 |
| Sodium Omadine | Na pyrithione | 500 | 1 | 16 |

The contribution to microbial inhibition supplied by each biocide in the mixture - Fractional Inhibitory Concentrations (FIC) - are calculated as the concentration of biocide which controlled growth in the mixture divided by the amount of biocide required to control growth when used alone. FIC values were calculated for all compounds under investigation in combination with the quaternary ammonium carbonate/bicarbonate.

**FIC = Concentration Biocide A attributable to antimicrobial activity in mixture / MIC of Biocide A**

The sum of these figures gives an indication of the action of the two biocides. A value less than one indicates a synergistic effect. If the total is equal to one, the action is additive or indifferent and if the value is greater than one the

biocides are antagonistic.

The results are shown below. The compositions in Tables 2, 5, and 11 refer to combinations of first and second biocide which are not part of the present invention.

**Synergistic Mixtures of Didecyl dimethyl ammonium carbonate/bicarbonate Against *Escherichia coli***

**Table 2:** (not according to the invention) **Fractional Inhibitory Concentrations Calculated for Didecyl dimethyl ammonium carbonate/bicarbonate and N-3-aminopropyl-N-dodecylpropane-1-3-diamine**

| FIC Didecyl dimethyl ammonium carbonate/bicarbonate | FIC N-3-aminopropyl-N-dodecylpropane-1-3-diamine | Total |
|---|---|---|
| 1.00 | 0.00 | 1 |
| 0.49 | 0.25 | 0.74 |
| 0.33 | 0.50 | 0.83 |
| 0.16 | 0.75 | 0.91 |
| 0.00 | 1.00 | 1.00 |

**Table 3: Fractional Inhibitory Concentrations Calculated for Didecyl dimethyl ammonium carbonate/ bicarbonate and Alkyldimethylbenzylammonium chloride**

| FIC Didecyl dimethyl ammonium carbonate/bicarbonate | FIC Alkyldimethylbenzylammonium chloride | Total |
|---|---|---|
| 1.00 | 0.00 | 1 |
| 0.67 | 0.25 | 0.91 |
| 0.33 | 0.50 | 0.83 |
| 0.16 | 0.75 | 0.91 |
| 0.00 | 1.00 | 1.00 |

**Table 4: Fractional Inhibitory Concentrations Calculated for Didecyl dimethyl ammonium carbonate/ bicarbonate and N,N-Didecyl-N-methyl-poly(oxyethyl)ammonium propionate**

| FIC Didecyl dimethyl ammonium carbonate/bicarbonate | FIC N,N-Didecyl-N-methyl-poly(oxyethyl) ammonium propionate | Total |
|---|---|---|
| 1.00 | 0.00 | 1 |
| 0.86 | 0.14 | 1.00 |
| 0.43 | 0.29 | 0.71 |
| 0.29 | 0.43 | 0.71 |
| 0.29 | 0.58 | 0.86 |
| 0.14 | 0.71 | 0.85 |
| 0.14 | 0.86 | 1.00 |
| 0.00 | 1.00 | 1.00 |

**Synergistic Mixtures of Didecyl dimethyl ammonium carbonate/bicarbonate Against *Pseudomonas aeruginosa***

**Table 5:** (not according to the invention) **Fractional Inhibitory Concentrations Calculated for Didecyl dimethyl ammonium carbonate/bicarbonate and N-3-aminopropyl-N-dodecylpropane-1-3-diamine**

| FIC Didecyl dimethyl ammonium carbonate/bicarbonate | FIC N-3-aminopropyl-N-dodecylpropane-1-3-diamine | Total |
|---|---|---|
| 1.00 | 0.00 | 1.00 |
| 0.60 | 0.33 | 0.93 |
| 0.20 | 0.67 | 0.87 |
| 0.00 | 1.00 | 1.00 |

**Table 6: Fractional Inhibitory Concentrations Calculated for Didecyl dimethyl ammonium carbonate/ bicarbonate and Alkyldimethylbenzylammonium** chloride

| FIC Didecyl dimethyl ammonium carbonate/bicarbonate | FIC Alkyldimethylbenzylammonium chloride | Total |
|---|---|---|
| 1.00 | 0.00 | 1.00 |
| 0.60 | 0.25 | 0.85 |
| 0.40 | 0.50 | 0.90 |
| 0.20 | 0.75 | 0.95 |
| 0.00 | 1.00 | 1.00 |

**Table 7: Fractional Inhibitory Concentrations Calculated for Didecyl dimethyl ammonium carbonate/ bicarbonate and Benzyl C12-16 alkyl dimethyl chlorides**

| FIC Didecyl dimethyl ammonium carbonate/bicarbonate | FIC Benzyl C12-16 alkyl dimethyl chlorides | Total |
|---|---|---|
| 1.00 | 0.00 | 1 |
| 0.55 | 0.35 | 0.90 |
| 0.27 | 0.65 | 0.92 |
| 0.00 | 1.00 | 1.00 |

**Table 8: Fractional Inhibitory Concentrations Calculated for Didecyl dimethyl ammonium carbonate/ bicarbonate and Alkyldimethylbenzylammonium chloride**

| FIC Didecyl dimethyl ammonium carbonate/bicarbonate | FIC Alkyldimethylbenzylammonium chloride | Total |
|---|---|---|
| 1.00 | 0.00 | 1 |
| 0.33 | 0.48 | 0.81 |
| 0.00 | 1.00 | 1.00 |

**Table 9: Fractional Inhibitory Concentrations Calculated for Didecyl dimethyl ammonium carbonate/ bicarbonate and N,N-Didecyl-N-methyl-poly(oxyethyl)ammonium propionate**

| FIC Didecyl dimethyl ammonium carbonate/bicarbonate | FIC N,N-Didecyl-N-methyl-poly(oxyethyl) ammonium propionate | Total |
|---|---|---|
| 1.00 | 0.00 | 1 |
| 0.33 | 0.35 | 0.69 |
| 0.33 | 0.65 | 0.98 |
| 0.00 | 1.00 | 1.00 |

**Table 10: Fractional Inhibitory Concentrations Calculated for Didecyl dimethyl ammonium carbonate/ bicarbonate and di C8-10 alkyl dimethyl chlorides + Benzyl C12-16 alkyl dimethyl chlorides**

| FIC Didecyl dimethyl ammonium carbonate/bicarbonate | FIC di C8-10 alkyl dimethyl chlorides + Benzyl C12-16 alkyl dimethyl chlorides | Total |
|---|---|---|
| 1.00 | 0.00 | 1 |
| 0.27 | 0.48 | 0.75 |
| 0.00 | 1.00 | 1.00 |

**Table 11:** (not according to the invention) **Fractional Inhibitory Concentrations Calculated for Didecyl dimethyl ammonium carbonate/bicarbonate and Na pyrithione**

| FIC Didecyl dimethyl ammonium carbonate/bicarbonate | FIC Na pyrithione | Total |
|---|---|---|
| 1.00 | 0.00 | 1 |
| 0.27 | 0.48 | 0.75 |
| 0.00 | 1.00 | 1.00 |

A synergistic interaction is the total effect of two chemicals being greater than their effects individually. In relation to disinfection, this means that a pair of synergistic biocides will have greater antimicrobial activity in the presence of each other against certain microorganisms in comparison to only a single biocide being present.

As shown above, dramatic and unexpected synergy was demonstrated between the quaternary ammonium carbonate/bicarbonate biocide in conjunction with the other biocides tested. In fact, as shown above, the sum of the fractional inhibitory concentrations of both biocides in many instances was less than about 0.9, such as less than about 0.8, such as less than about 0.7. The sum of the fractional inhibitory concentrations of the two biocides was generally greater than about 0.4.

## Claims

**1.** A composition having biocidal properties comprising:

a first biocide comprising a salt of a quaternary ammonium cation, the first biocide comprising a carbonate/bicarbonate salt of a quaternary ammonium cation; and

a second biocide selected so as to synergistically operate in conjunction with the first biocide in order to kill or inhibit growth of a microorganism, the second biocide comprising an alkyl quaternary ammonium halide, a benzyl ammonium halide, or a quaternary ammonium propionate, and wherein the second biocide is present in relation to the first biocide such that the ability of both biocides to kill or inhibit the growth of the microorganism is greater than if only one of the biocides were present at the same concentration of both biocides together;

wherein the first biocide and the second biocide are present in the composition at a weight ratio of from about 1:20 to about 20:1, wherein

the first biocide is a didecyl dimethyl ammonium carbonate/bicarbonate, and wherein

the second biocide is a benzyl alkyl dimethyl ammonium chloride and/or an N,N-didecyl-N-methyl-poly(oxyethyl) ammonium propionate.

**2.** A composition as defined in claim 1, wherein the second biocide comprises a benzyl $C_{12}$ to $C_{18}$ alkyl dimethyl ammonium chloride.

**3.** A composition as defined in any of the preceding claims, wherein the first biocide and the second biocide are present in the composition at a weight ratio of from about 1:5 to about 5:1.

**4.** A disinfectant composition comprising the composition according to claims 1-3 and one or more aids selected from the group comprising solvents, surfactants, sequestering agents, complexing agents, corrosion inhibitors, alkalinity builders, pH adjusting agents, preservatives, auxiliaries, acids, foaming agents, perfumes, and colorants.

**5.** A cosmetic or hygiene product comprising:

a base formulation; and
a preservative comprising a composition according to any of claims 1-3.

**6.** A method for disinfecting a hard surface comprising:
applying the composition having biocidal properties as defined in any of claims 1-4 to a hard surface.

**7.** Non-therapeutic use of a composition according to claim 1, for killing or inhibiting the growth of microorganisms, wherein the microorganism comprises a gram positive bacteria, a gram negative bacteria, a fungi, a virus, a yeast, a spore, or a mycobacteria.

**8.** Non-therapeutic use according to claim 7, wherein the microorganism comprises *Staphylococcus aureus, Streptococcus pneumoniae, Pseudomonas aeruginosa, Serratia marcescens, Salmonella enteritidis, Neisseria gonorrhoeae, Escherichia coli, Enterococcus hirae, Acinetobacter baumannii, Listeria monocytogenes, Enterobacter gergoviae, Klebsiella pneumoniae, Burkholderia cepacia, Pseudomonas putida, Kocuria rhizophila, Candida albicans, Saccharomyces cerevisiae, Aspergillus brasiliensis, Penicillium funiculosum, Eupenicillium levitum, Bacillus cereus, Bacillus subtilis, Clostridium difficile, Clostridium perfringens, Mycobacterium tuberculosis, Mycobacterium terrae, Mycobacterium avium,* Poliovirus, Adenovirus, Norovirus, Vaccinia virus, Influenza virus, Hepatitis B virus, Human Immunodeficiency virus, and/or Human papilloma virus

## Patentansprüche

**1.** Zusammensetzung mit bioziden Eigenschaften, die aufweist:

ein erstes Biozid, das ein Salz eines quartären Ammoniumkations aufweist, wobei das erste Biozid ein Carbonat-/Bicarbonat-Salz eines quartären Ammoniumkations aufweist; und
ein zweites Biozid, das derart ausgewählt ist, dass es synergistisch in Verbindung mit dem ersten Biozid wirkt, um einen Mikroorganismus abzutöten oder dessen Wachstum zu hemmen, wobei das zweite Biozid ein quartäres Alkylammoniumhalogenid, ein Benzylammoniumhalogenid oder ein quartäres Ammoniumpropionat aufweist und wobei das zweite Biozid in Bezug auf das erste Biozid derart vorliegt, dass die Fähigkeit beider Biozide, den Mikroorganismus abzutöten oder dessen Wachstum zu hemmen, größer ist, als wenn nur eines der Biozide in einer Konzentration vorliegen würde, die der Gesamtkonzentration beider Biozide entspricht;
wobei das erste Biozid und das zweite Biozid in der Zusammensetzung in einem Gewichtsverhältnis von etwa 1:20 bis etwa 20:1 vorliegen, wobei
das erste Biozid ein Didecyldimethylammoniumcarbonat/-bicarbonat ist und wobei
das zweite Biozid ein Alkylbenzyldimethylammoniumchlorid und/oder ein N,N-Didecyl-N-methyl-poly(oxyethyl) ammoniumpropionat ist.

**2.** Zusammensetzung nach Anspruch 1, wobei das zweite Biozid ein $C_{12}$- bis $C_{18}$-Alkylbenzyldimethylammoniumchlorid aufweist.

**3.** Zusammensetzung nach einem der vorhergehenden Ansprüche, wobei das erste Biozid und das zweite Biozid in der Zusammensetzung in einem Gewichtsverhältnis von etwa 1:5 bis etwa 5:1 vorliegen.

**4.** Desinfektionsmittel-Zusammensetzung, die die Zusammensetzung nach den Ansprüchen 1-3 und einen oder mehrere Hilfsstoffe aufweist, der ausgewählt ist aus der Gruppe, die Lösungsmittel, Tenside, Sequestrierungsmittel, Komplexierungsmittel, Korrosionsinhibitoren, Alkalinitätsbildner, pH-Einstellmittel, Konservierungsmittel, Hilfsmittel, Säuren, Schäummittel, Parfüme und Farbstoffe umfasst.

**5.** Kosmetik- oder Hygieneprodukt, das aufweist:

eine Grundformulierung; und
ein Konservierungsmittel, das die Zusammensetzung nach einem der Ansprüche 1-3 aufweist.

**6.** Verfahren zum Desinfizieren einer harten Oberfläche, das umfasst:
Aufbringen der Zusammensetzung mit bioziden Eigenschaften nach einem der Ansprüche 1-4 auf eine harte Oberfläche.

7. Nichttherapeutische Verwendung der Zusammensetzung nach Anspruch 1 zum Abtöten oder Hemmen des Wachstums von Mikroorganismen, wobei der Mikroorganismus ein grampositives Bakterium, ein gramnegatives Bakterium, einen Pilz, ein Virus, eine Hefe, eine Spore oder ein Mykobakterium umfasst.

8. Nichttherapeutische Verwendung nach Anspruch 7, wobei der Mikroorganismus *Staphylococcus aureus, Streptococcus pneumoniae, Pseudomonas aeruginosa, Serratia marcescens, Salmonella enteritidis, Neisseria gonorrhoeae, Escherichia coli, Enterococcus hirae, Acinetobacter baumannii, Listeria monocytogenes, Enterobacter gergoviae, Klebsiella pneumoniae, Burkholderia cepacia, Pseudomonas putida, Kocuria rhizophila, Candida albicans, Saccharomyces cerevisiae, Aspergillus brasiliensis, Penicillium funiculosum, Eupenicillium levitum, Bacillus cereus, Bacillus subtilis, Clostridium difficile, Clostridium perfringens, Mycobacterium tuberculosis, Mycobacterium terrae, Mycobacterium avium,* ein Poliovirus, ein Adenovirus, ein Norovirus, ein Vacciniavirus, ein Influenzavirus, ein Hepatitis-B-Virus, ein Humanes Immundefizienz-Virus und/oder ein Humanes Papillomvirus umfasst.

## Revendications

1. Composition ayant des propriétés biocides comprenant:

   un premier biocide comprenant un sel d'un cation ammonium quaternaire, le premier biocide comprenant un sel carbonate/bicarbonate d'un cation ammonium quaternaire; et
   un deuxième biocide choisi de manière à agir en synergie avec le premier biocide afin de tuer ou d'inhiber la croissance d'un micro-organisme, le deuxième biocide comprenant un halogénure d'alkylammonium quaternaire, un halogénure de benzylammonium, ou un propionate d'ammonium quaternaire, et dans lequel le deuxième biocide est présent par rapport au premier biocide de telle sorte que la capacité des deux biocides à tuer ou à inhiber la croissance du micro-organisme est plus grande que si un seul des biocides était présent à la même concentration des deux biocides ensemble;
   dans laquelle le premier biocide et le deuxième biocide sont présents dans la composition dans un rapport pondéral d'environ 1:20 à environ 20:1, dans laquelle
   le premier biocide est un carbonate/bicarbonate de didécyl diméthyl ammonium, et dans laquelle
   le deuxième biocide est un chlorure de benzylalkyl diméthyl ammonium et/ou un propionate de N,N-didécyl-N-méthyl-poly(oxyéthyl)ammonium.

2. Composition selon la revendication 1, dans laquelle le deuxième biocide comprend un chlorure de diméthylammonium alkyl benzylique en $C_{12}$ à $C_{18}$.

3. Composition selon l'une quelconque des revendications précédentes, dans laquelle le premier biocide et le deuxième biocide sont présents dans la composition dans un rapport pondéral d'environ 1:5 à environ 5:1.

4. Composition désinfectante comprenant la composition selon les revendications 1-3 et un ou plusieurs auxiliaires choisis dans le groupe comprenant des solvants, des tensioactifs, des agents séquestrants, des agents complexants, des inhibiteurs de corrosion, des agents augmentant l'alcalinité, des agents correcteurs de pH, des agents de conservation, des auxiliaires, des acides, des agents moussants, des parfums, et des colorants.

5. Produit cosmétique ou d'hygiène comprenant:

   une formulation de base; et
   un agent de conservation comprenant une composition selon l'une quelconque des revendications 1 à 3.

6. Procédé de désinfection d'une surface dure comprenant:
   l'application sur une surface dure de la composition ayant des propriétés biocides telles que définies dans l'une quelconque des revendications 1-4.

7. Utilisation non thérapeutique d'une composition selon la revendication 1, pour tuer ou inhiber la croissance de micro-organismes, dans laquelle le micro-organisme comprend une bactérie à Gram positif, une bactérie à Gram négatif, un champignon, un virus, une levure, une spore, ou une mycobactérie.

8. Utilisation non thérapeutique selon la revendication 7, dans laquelle le micro-organisme comprend *Staphylococcus aureus, Streptococcus pneumoniae, Pseudomonas aeruginosa, Serratia marcescens, Salmonella enteritidis, Neis-*

*seria gonorrhoeae, Escherichia coli, Enterococcus hirae, Acinetobacter baumannii, Listeria monocytogenes, Enterobacter gergoviae, Klebsiella pneumoniae, Burkholderia cepacia, Pseudomonas putida, Kocuria rhizophila, Candida albicans, Saccharomyces cerevisiae, Aspergillus brasiliensis, Penicillium funiculosum, Eupenicillium levitum, Bacillus cereus, Bacillus subtilis, Clostridium difficile, Clostridium perfringens, Mycobacterium tuberculosis, Mycobacterium terrae, Mycobacterium avium,* poliovirus, adénovirus, norovirus, virus de la vaccine, virus de la grippe, virus de l'hépatite B, virus de l'immunodéficience humaine, et/ou virus du papillome humain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62354425
- US 2005053516 A1
- CA 1164338 A
- WO 2011116775 A2
- WO 0065911 A1
- US 6080789 A
- WO 9512976 A1
- US 2015282480 A1
- US 2009023790 A1
- JP H02184604 A


### Non-patent literature cited in the description


- **LONZA**. *BARDAC (TM) 205M BARDAC (TM) 208M Quaternary Ammonium Compounds EPA Registered*, 29 July 2007, 1-2, http://bio.lonza.com/uploads/tx_mwaxmarketingmaterial/Lonza_ProductDataSheet s_Bardac_208M_PDS.pdf and WEIGERT
- *Neodisher Dekonta AF - technical information*, 1-2, https://www.drweigert.com/de/uploads/tx_product_-manager/downloads/product/dat aSheet/neodisher-Dekonta-AF_MB_de_PN4018_2015-05-01.pdf
- **PONS J-L et al.** EVALUATION OF ANTIMICROBIAL INTERACTIONS BETWEEN CHLORHEXIDINE, QUATERNARY AMMONIUM COMPOUNDS, PRESERVATIVES AND EXCIPIENTS. *JOURNAL OF APPLIED BACTERIOLOGY*, 01 January 1992, vol. 73, 395-400
- *CHEMICAL ABSTRACTS*, 1121-31-9
- *CHEMICAL ABSTRACTS*, 1121-30-8